# EUROPEAN PATENT SPECIFICATION

(11) **EP 2 334 447 B1**
(45) Date of publication and mention of the grant of the patent: **17.03.2021**
(21) Application number: 09740841.3
(22) Date of filing: 15.09.2009
(51) Int. Cl.: A61L 2/26, B08B 13/00, A61G 7/05, B08B 3/00, A61L 2/04

(54) **HOSPITAL AND CARE FURNITURE WITH AN EQUIPMENT FOR SERVICING AND METHOD OF SERVICING HOSPITAL OR CARE FURNITURE**
KRANKENHAUS- UND PFLEGEMÖBELN MIT EINRICHTUNG FÜR DIE INSTANDHALTUNG
ÉQUIPEMENT POUR L'ENTRETIEN D'UN MOBILIER D'HÔPITAL ET DE SOINS

(30) Priority: 15.09.2008 DK 200801297
(43) Date of publication of application: 22.06.2011
(73) Proprietor: Linak A/S, 6430 Nordborg (DK)
(72) Inventor: JENSEN, Peter, Brøndum, DK-6400 Sønderborg (DK); JENSEN, Svend, Erik, Knudsen, DK-7100 Vejle (DK)
(74) Representative: Høyer, Michael
(86) International application number: PCT/DK2009/000203
(87) International publication number: WO 2010/145654

(56) References cited:
- WO-A1-2009/033487
- WO-A2-2006/109050
- DE-A1-102007 039 088
- US-A- 5 359 993
- US-A1- 2005 183 656
- US-A1- 2007 210 917

## Description

The invention relates to equipment for use in servicing of hospital and care furniture, e.g. a hospital or care bed, a patient lifter, a treatment table, a chair as defined in the preamble of claim 1. Further, the invention relates to a method for use in servicing of hospital and care furniture based on the cleaning of these.

Hospital and care beds are fairly complicated structures both mechanically and electrically and require thorough servicing. An example of such a bed is known from US 6,752,224 B2 Stryker Corporation. In hospitals and nursing homes the beds are thus regularly sent to be serviced.

Typically the beds, i.e. bed frame with framework and system for adjusting the bed, is transported to a central cleaning department and driven through a washer apparatus, which is constructed specially for cleaning articles of hospital- and care furniture. Such a washer apparatus is e.g. known from FR 2 744 041 to Athen

WO2009/033487A1 to LINAK A/S discloses a system that alerts a need for cleaning of the hospital or care bed. The trigger of the alert is the time that passes since last cleaning and the number of adjustments of the profile of the bed.

WO2006/109050A2 to SMARTA SYSTEMS LTD discloses a monitoring system for an adjustable furniture, which can be configured to alert that service is needed.

DE102007039088A1 to DRAEGER MEDICAL AG discloses an apparatus for cleaning, which facilitates a temperature measurement and a counter for counting the number of thermocycles performed.

US2005/0183656A1 to Isaacson et al discloses a surgical sterilization device, which features a counter for counting the number of operations performed and alerting when exceeding a predefined number of operations.

US5,359,993 to SYMBIOSIS CORPORATION discloses a medical instrument with a mechanical counter for counting the number of times the medical instrument has been used.

US2007/210917A1 to Collins Williams F JR et al discloses a monitoring system for monitoring a bed and transmitting the data to a monitoring post.

Cleaning of the furniture is a necessary measure, which however has the disadvantage that it has a negative impact on the mechanical state of the bed. It is e. g. inevitable that the lubricant over time will be degraded or washed away when the piece of furniture is washed in a washer apparatus.

The requirement for service after cleaning is intensified when using stronger cleaning agents. It is not uncommon that highly acid or alkaline cleaning agents are used, which can ruin seals in the electric actuators or housings for electric controls, so that these are no longer waterproof. At the same time lubricants in the electric drives or in the mechanical connections on the piece of furniture are degraded or washed away.

The purpose of the invention is to provide a solution to the outlined problem, which is to ensure timely servicing of hospitals- and care beds.

This is achieved according to the invention with the solution as stated in claim 1.

Here, it is stated that the equipment comprises a counter unit, which counts number of cleanings of the article of furniture in order to indicate when the article of furniture again needs servicing optionally a timer unit which defines a period of time in which the article of furniture again needs servicing. However, expediently it is a combination of a counter and a timer unit. Thus it is ensured that the article of furniture is serviced within a given period of time even though it has not been cleaned the expected number of times within the given time period. E.g. if cleaning is expected twenty-five times a year and that number of cleanings is not attained, it is nevertheless ensured that the article of furniture is serviced at least once a year.

By introducing a counter into the equipment which counts the number of performed cleanings, the equipment will be able to indicate a need for servicing after a predetermined number of cleanings. The required servicing may thus be carried out after which the indicator may be reset.

The signaling device which gives a signal, when the piece of furniture has been cleaned may be a simple mechanical switch which is operated when the article of furniture is driven out of the washer apparatus. It may also be a switch on an operation unit or a sensor e.g. a RFID or a magnetic sensor.

In the invention, the equipment is furnished with a thermo sensor which gives a signal, when the temperature exceeds a threshold value. In that way, it is possible to automatically register that the piece of furniture is undergoing cleaning in a washer apparatus. The signal is used for automatically incrementing the counter in the equipment, which counts the number of cleanings of the article of furniture. The signal from the thermo sensor is practically constructed with a hysteresis so that the signal does not disappear before the temperature has dropped considerably, e.g. to below approximately 40°C, usually combined with a period of time which also has to expire. This prevents that a cleaning of the article of furniture is interpreted as more than one count on the counter and thus ensures that the correct number of cleanings of the article of furniture is registered.

When the article of furniture is being serviced the counter for servicing will have to be reset, which expediently may be performed by means of a reset key. To avoid unintended resetting of the service indication, a combined signal may be used which consists of pressing a key at the same time as activating a Hall sensor by moving a magnet in its presence. The Hall-sensor is connected to the microprocessor which processes the signals from the keys and the Hall-sensor, so that the microprocessor may reset the counter. The communication with the microprocessor and other units in connection with the article of furniture includes being able to externally control and reset the counter for indication of service.

The position of the Hall-sensor at one of the sides in the housing should then either be known or indicated on the housing.

The indication of the requirement for service can be visually presented on a panel of light-emitting diodes, where the simplest embodiment is a light-emitting diode, which is switched-on when service is required. The panel can be expanded so that more light-emitting diodes show the status of the process, so that it beforehand can be chosen to perform servicing if it fits the time table better. In more advanced solutions, displays can be used, e.g. seven segment digit display or graphic displays with text and figures. The indicators can be located in a housing which subsequently can be mounted on the piece of furniture or in a control unit for adjusting the piece of furniture. It is also possible to communicate the status on to a central operator terminal located at a distance from the article of furniture.

The requirement for service after cleaning is intensified when using stronger cleaning agents. It is not uncommon that highly acid or alkaline cleaning agents are used, which can ruin seals in the electric actuators or housings for electric controls, so that these are no longer waterproof. At the same time lubricants in the electric drives or in the mechanical connections on the piece of furniture are degraded or washed away. It is therefore an advantage if the equipment is also furnished with an instrument for measuring the pH value during washing and give a signal for accelerating servicing of the article of furniture if aggressive cleaning agents are used for the cleaning. The measuring instrument can be located outside the housing and be connected via a cable or be positioned in the housing. The signal from the pH sensor as input for the counter for the number of cleanings means that the number of cleanings to be performed between servicing can vary from a determine formula or table, depending on the intensity of the cleaning of the piece of furniture.

For communication between the equipment for use in connection with cleaning of articles of hospital and care furniture and the control, which manages the adjustment of the bed, the communication system "Openbus" according to WO2007/057014 to Linak is used. Openbus describes a standard for communication between the various units attached to the article of furniture, this being hand control(s), the individual actuator, etc. It is therefore possible to communicate various parameters between the control and the individual Openbus-connected unit. The equipment can via a gateway be connected to networks in the hospital which can comprise a central monitoring post, operated by the nursing staff and/or staff in charge of maintenance and cleaning.

With "Openbus" is provided several possibilities for communication of status of the piece of furniture including duplex communication directly with the equipment. It will thus be possible to transfer the indication "service required" with various parameters as to what needs servicing, directly to the central monitoring. The sorting of the indication can take place so that a request for servicing is sent directly to the maintenance staff or the hospital porter, who is in charge of collecting the piece of furniture and bringing it to servicing. When it comes to external companies which take care of maintenance, an indication of service or repair of the article of furniture could e.g. be sent via the internet from the monitoring post.

If the monitoring post is connected to the database containing data about the article of furniture, it will from the monitoring post, based on the state of the article of furniture, be possible to program the parameters of the equipment, so that a request for intensified servicing e.g. will trigger an indication of "servicing required" earlier than, what is otherwise standard for the equipment. When a patient is discharged, it will be possible from the monitoring post manually or automatically to externally control the equipment to show "servicing required" and thus ensuring that the piece of furniture will be serviced before the next patient occupies it.

The invention also relates to a method for servicing of hospital and care furniture according to claim 14. In a specific embodiment this method includes an initiation or reset state, an operating condition with indication of the status and an alarm condition, where the initiation state is carried out manually by activating with a sensor, preferably a Hall-sensor with a magnet, and resets a counter, which counts the number of cleanings and further updates the status on an indication panel and where the operating condition maintains a counter which counts the number of cleanings of the article of furniture and as input has a sensor, preferably a pH sensor, which measures the aggressiveness of the cleaning agent and manipulates the counter with a factor, corresponding to the extra need for service and where the alarm condition occurs when the counter, which counts the number of cleanings of the article of furniture exceeds a determined number, where the alarm condition is indicated on an indication panel.

The invention may either be carried out as a separate unit or as an extension to the existing control, which manages the adjustment of the article of furniture.

The equipment used in the invention will be described more fully below with reference to the accompanying drawing, in which
Fig. 1, shows a hospital bed in a washer apparatus,
Fig. 2, shows a hospital bed having the equipment mounted thereon,
Fig. 3, shows a flow chart of the equipment, and
Fig. 4, shows a housing for the equipment.

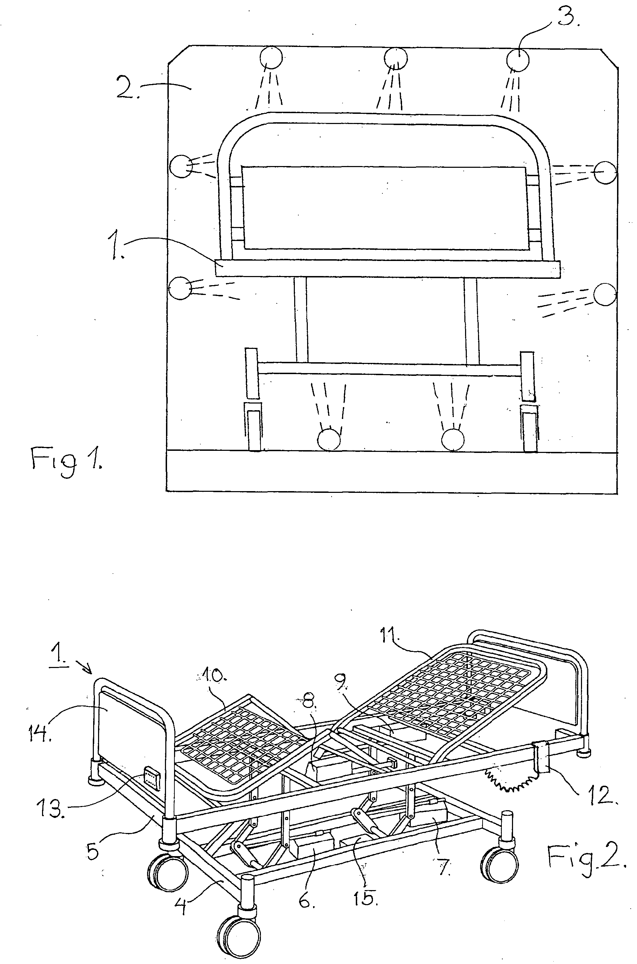

Fig. 1 of the drawing shows a hospital bed 1 during cleaning in a washer apparatus 2 specially constructed for cleaning hospital beds 1. The washer apparatus 2 is constructed with a number of jets 3 through which the cleaning agent and water is injected into the chamber of the washer apparatus and thus efficiently cleaning the hospital bed.

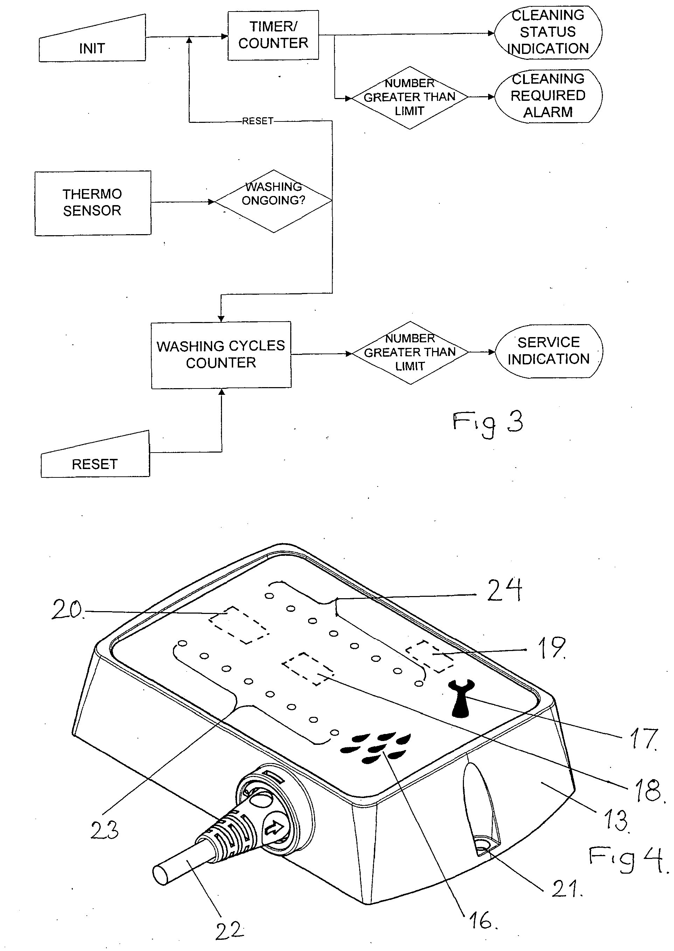

The hospital bed 1 is further shown in Fig. 2, where it is apparent that it is constructed with a lower frame 4 and a an upper frame 5, which are height-adjustable in proportion to each other by activation of the electric linear actuators 6, 7. The electric linear actuators 8, 9 serve to adjust the support surfaces 10,11. The adjustment is carried out by operating a hand control 12. The equipment 13 is shown as a separate unit mounted on a bed end 14 at the foot end of the bed. The equipment incorporated in its own housing is incidentally shown in Fig. 4. Even though the equipment here is shown in its own housing, the equipment could also be integrated in the control box 15 for the actuators comprising the control. The flow chart in Fig. 3 shows that the system is initiated when the bed has been cleaned, after which a timer process and/or counter process is started. On an indication panel, the status of the equipment is shown so that it becomes apparent how much time has elapsed so far, and is left until the bed needs cleaning. A counter is started parallel to the process and counts the number of operations of the actuator system. When the timer and/or the counter reaches a threshold value, the indication that the bed needs cleaning is started. The system can be reset either by operating the keys 16, 17 of the panel in a certain sequence or by means of a switch on the bed, which can be activated manually. It could e.g. be a key operated switch or alternatively a magnetic switch in connection with the bed, which can be activated by means of a magnet located in the washer apparatus. In a special embodiment, the equipment is furnished with a thermo sensor 18 which gives a signal when the temperature exceeds a threshold value, e.g. over 50°C. In that way, it is possible to automatically register that the bed is undergoing cleaning in a washer apparatus 2. The signal can be used for automatically resetting the timer/counter so that a new timer/counter period can elapse before the equipment again raises the alarm. The signal from the thermo sensor 18 is practically constructed with a hysteresis so that the signal does not disappear before the temperature has dropped considerably, e.g. below 40°C. It may if convenient be combined with a period of time which also must expire. This prevents the signal from fluctuating and is important in connection with the wish for registering the number of cleanings of the bed. The number of performed cleanings can be used statistically, but also to estimate when the bed should be serviced. It is especially practical to establish a counter, which registers the number of performed cleanings, so that a number of cleanings triggers an indication that servicing of the bed is required. Such an indication should be able to be reset, but only by authorized staff. For that reason a magnetic sensor 19 is built into the housing, so that it is possible to place a magnet outside the housing in its vicinity to be able to perform a reset of the indication. Practically, the reset function would be able to discontinue a simultaneous key combination, so that unintended operation is avoided. As the service interval is not only determined by the number of cleanings of the bed, but also of the strength of the cleaning agent, a pH sensor 20 is introduced, to provide possibility for accelerating servicing if this is required. A formula or table in the microprocessor of the control may be constructed so that the change in the service interval precisely can be determined given the aggressiveness of the cleaning agents being used to clean the bed.

As it appears from Fig. 4 of the drawing the equipment is built into its own housing 13 and appears in this embodiment as a separate unit. On the housing there is a section 21 with holes for attachment of the housing on the bed, typically on the bed frame 5 or the bed end 14. The housing is supplied with power via the cable 22 mounted on the housing 13. On the front of the housing, the switches and indicators are placed and protected by a fully covering foil. The pictograms for cleaning or servicing thereby show where the switches 16, 17 are located. The equipment is initiated by pressing the switch 16 for a certain duration, typically a few seconds. This takes place to avoid unintended initiation of the equipment, which possibly could happen if unauthorized persons activate the switch briefly. When the equipment is initiated, the cleaning indication panel 23, which consists of a row of light-emitting diodes, will return to the initial position and one indicator is switched-on. At the same time, a timer process starts, which expires when the bed needs cleaning. Proportional to the timer process, more and more indicators 23 are switched on, for then eventually at the expiration of the timer process, having all indicators switched-on. Alternatively, one switched-on light-emitting diode indicates status for the process by its position in the row.

The equipment is also furnished with a thermo sensor 18, located directly against the front of the housing 13, for measuring the surrounding temperature of the bed. An increase in temperature can then indicate that the bed is in the washer apparatus 2 and is being cleaned. The signal is also used for resetting the equipment so that a new timer/counter period can start. The number of cleanings is summed up by a counter for indicating a need for service and is indicated on the service indication panel 24, which consists of a row of light-emitting diodes. When a preset number of cleanings has been carried out, an indication of the need for service will appear. The need for service can be accelerated by use of stronger cleaning agents, so in order to register this, a pH-sensor 20 is connected to the housing, It is possible to connect an external pH-sensor or to build a pH-sensor 20 directly into the housing. In the drawing, a possibility for the positioning is shown, but other positions can also be anticipated. Input from the pH-sensor during washing will be used to attribute the counter for the service indicator a factor, so a more thorough cleaning means that the counter reaches the threshold where a need for service is indicated earlier.

The indication of the need for service is reset in that a magnetic object is brought within reach of a Hall-sensor 19 located directly against the front of the housing 13. It is practical that the resetting besides from the activation of the Hall-sensor 19 with a magnet involves a pressing of the key 17 or a combination of keys so that unintended resetting is avoided.

The cable 22 comprises cords for power supply but also a connection to a potential extern initiation switch located on the bed 1. If the bed 1 is cleaned in an automatic washer apparatus 2 for beds, the washer apparatus may be equipped with an arrangement, which activates the initiation switch, which can be a conventional switch or a switch based on a sensor, e.g. a magnetic sensor or e.g. RFID which allows duplex communication.

In the cable 22 there is also an interface for the control 15 of the bed. In this way there is access to other parameters in the use of the bed, which can be used for determining if the bed 1 needs cleaning. E.g. the number of activations of the hand control 12 is used as a supplementary input, which can trigger a request for cleaning before the expiration of the timer process. The signal, which is transferred to the control 15, is sophisticated so to understand, that the requirement for cleaning can be attributed to the individual elements in the bed: the bed frame with adjustable framework 4,5,10,11, control 15, electric actuators 6,7,8,9 and control unit 13; the mattress of the bed; the bed linen and pillow; control units as independent units. It is therefore given that different time periods can be programmed for indication of request for cleaning for the various elements of the bed.

The equipment is microprocessor based and can thus be programmed to an indication according to the wishes of the customer. The programming is carried out from a PC with specially tailored software connected to the control via a cable. The cable is connected to a bus-connection on the microprocessor, which is lead to a plug and socket connection on the printed circuit board. Because of this programmable flexibility, it will, as a part of the invention, be possible to differentiate from the standard solution in that an indicator shows the reason that the requirement for cleaning has been triggered. This can be done by directly dedicating one or more of the light-emitting diodes in the indication panel 23 to this purpose. If it is chosen to let only one indicator serve this purpose, the frequency, with which the indicator blinks or the color of this, could be used to indicate, what has triggered the requirement for cleaning. That a LED panel is described here to indicate the need for cleaning, does not exclude that another type of display can be used, e.g. a LCD. A display can also have digits like e.g. a seven segment display or a graphic display. The same flexible structure is used in the indicator panel 24 for indication of the need for service, where the appearance of each light-emitting diode can be programmed separately.

For communication between the equipment for use in connection with cleaning of articles of hospital and care furniture and the control, which manages the adjustment of the bed, the communication system "Openbus" according to WO2007/057014 to Linak is used. Openbus describes a standard for communication between the various units attached to the bed, this being hand control (s), the individual actuator, etc. It is therefore possible to communicate various parameters between the control and the individual Openbus-connected unit. The control can via a gateway be connected to network in the hospital, which can comprise a central monitoring post, operated by the nursing staff and/or staff in charge of maintenance and cleaning. With "Openbus" is provided several possibilities for communication of status of the bed including duplex communication directly with the equipment for use in connection with cleaning of articles of hospital and care furniture. It will thus be possible to transfer the indication "cleaning required" or "servicing required" with various parameters as to what needs cleaning or servicing, directly to the central monitoring. The sorting of the indication can take place so that a request for cleaning is sent directly to the cleaning staff or the hospital porter, who is in charge of collecting the bed and bringing it to cleaning. When it comes to external companies which take care of maintenance, an indication of service or repair could e.g. be sent via the internet from the monitoring post. If the monitoring post is connected to the database containing data about registered patients, it will from the monitoring post based on the hygienic condition of the patient be possible to program the parameters of the equipment, so that a request for intensified hygiene e.g. will trigger an indication of "cleaning required" earlier than, what is otherwise standard for the equipment. When a patient is discharged, it will be possible from the monitoring post manually or automatically to externally control the equipment for use in connection with cleaning of hospital and care furniture to show "cleaning required" and/or "servicing required", and thus ensuring that the bed will be cleaned and serviced before the next patient occupies it.

## Claims

1. Piece of hospital or care furniture selected from the group of: a hospital or care bed, a patient lifter, a treatment table, a chair, where the piece of hospital or care furniture comprises:
- at least one electric linear actuator (6, 7, 8, 9) for adjusting the piece of furniture (1),
- at least one hand control unit (12)
- at least one control (15) comprising a microprocessor **characterized in that** the piece of hospital or care furniture comprises an equipment comprising:
- a counter unit and
- a signaling device,
where the signaling device being configured to provide a signal indicating that the piece of furniture (1) has been cleaned is a thermo sensor (18) which gives a signal when the temperature of the equipment exceeds a threshold value, by which signal the counter is automatically incremented and when a predetermined number of counts is reached, the counter unit will indicate a need for service.

2. Piece of hospital or care furniture according to claim 1, **characterized in that** the signal indicating that the piece of furniture has been cleaned is constructed with a hysteresis so that the signal does not disappear before the temperature has dropped considerably combined with a time that must expire.

3. Piece of hospital or care furniture according to claim 1, **characterized in that** the equipment is a separate unit or an extension to the control (15) which manages the adjustment of the piece of furniture.

4. Piece of hospital or care furniture according to claim 1, **characterized in that** the counter unit counting the number of cleanings of the piece of furniture (1) is equipped with a device for resetting of the counter unit.

5. Piece of hospital or care furniture according to claim 4, **characterized in that** the device for resetting of the counter unit is a magnet-based switch, which is activated by placing a magnet in its presence.

6. Piece of hospital or care furniture according to claim 5, **characterized in that** the magnet-based switch is a Hall-sensor (19).

7. Piece of hospital or care furniture according to claim 4 or 6, **characterized in that** the device for resetting the counter unit further involves a pressing of a key (17) or pressing of a combination of keys.

8. Piece of hospital or care furniture according to claim 4, **characterized in that** the device for resetting the counter unit is activated through communication with the microprocessor.

9. Piece of hospital or care furniture according to claim 1, **characterized in that** the indication that the piece of furniture (1) needs servicing has been visualized by means of indicators (24) constructed as a LED panel e.g. located in a housing (13) mounted on the piece of furniture (1).

10. Piece of hospital or care furniture according to claim 1, **characterized in that** the equipment (13) is furnished with a measuring apparatus (20) for measuring the pH value of the cleaning agent during washing.

11. Piece of hospital or care furniture according to claim 10, **characterized in that** the equipment (13) based on pH-value of the cleaning agent during washing calculates a factor which, together with the number of cleanings of the piece of furniture since the last servicing of the piece of furniture (1), forms part of the determination of when an indication of required servicing of the piece of furniture (1) appears.

12. Piece of hospital or care furniture according to claim 1, **characterized in that** the equipment (13) is furnished with a timer unit which indicates when the piece of furniture again needs servicing.

13. Piece of hospital or care furniture according to any of the preceding claims, **characterized in that** the equipment (13) has means for exchanging data with other units in the piece of furniture (1), where other units could be one of the following:
a. a control (15),
b. a gate way, or
c. a hand control (12)
and where the data to be communicated could be:
d. the number of activations of control units,
e. status for timer or counting circuit,
f. status for thermo sensor,
g. reset or initiating of timers or counters,
h. set-up timer or counter thresholds,
i. the number of cleanings since the last servicing,
j. configuration of displaying on indication panel,
k. configuration of functions on keys,
1. configuration of routine of calculation of pH measuring,
m. external control of timers, counters or indicators.

14. Method for servicing of hospital and care furniture selected from the group of: a hospital or care bed, a patient lifter, a treatment table, a chair, based on the cleaning of these, including an initiation or reset state, an operating condition with indication of the status and an alarm condition,
where the initiation state is carried out manually by activating a sensor, preferably a Hall-sensor with a magnet, and resets a counter, which counts the number of cleanings and further updates the status on an indication panel, and
where the operating condition maintains the counter, which counts the number of cleanings of the piece of furniture and
where the alarm condition occurs when the counter, which counts the number of cleanings of the piece of furniture exceeds a determined number, where the alarm condition is indicated on an indication panel.

15. Method for servicing of hospital and care furniture according to claim 14 where the hospital and care furniture as input has a pH sensor, which measures the aggressiveness of the cleaning agent and manipulates the counter with a factor, corresponding to the extra need for service.

## Patentansprüche

1. Krankenhaus- oder Pflegemöbelstück, ausgewählt aus der Gruppe: ein Krankenhaus- oder Pflegebett, ein Patientenlifter, ein Behandlungstisch, ein Stuhl, wobei das Krankenhaus- oder Pflegemöbelstück aufweist:
- mindestens einen elektrischen Linearantrieb (6, 7, 8, 9) zum Verstellen des Möbelstücks (1),
- mindestens eine Handsteuereinheit (12),
- mindestens eine Steuerung (15) mit einem Mikroprozessor,
**dadurch gekennzeichnet, dass** das Krankenhaus- oder Pflegemöbelstück eine Ausstattung aufweist, die umfasst:
- eine Zählereinheit und
- eine Signalisierungseinrichtung,
wobei die Signalisierungseinrichtung, die so konfiguriert ist, dass sie ein Signal liefert, das anzeigt, dass das Möbelstück (1) gereinigt wurde, ein Thermosensor (18) ist, der ein Signal abgibt, wenn die Temperatur der Ausstattung einen Schwellenwert überschreitet, wobei durch dieses Signal der Zähler automatisch erhöht wird und wenn eine vorbestimmte Anzahl von Zahlern erreicht ist, die Zählereinheit einen Wartungsbedarf anzeigt.

2. Krankenhaus- oder Pflegemöbelstück nach Anspruch 1, **dadurch gekennzeichnet, dass** das Signal, das anzeigt, dass das Möbelstück gereinigt worden ist, mit einer Hysterese konstruiert ist, so dass das Signal nicht verschwindet, bevor die Temperatur erheblich gesunken ist, verbunden mit einer Zeit, die ablaufen muss.

3. Krankenhaus- oder Pflegemöbelstück nach Anspruch 1, **dadurch gekennzeichnet, dass** die Ausrüstung eine separate Einheit oder eine Erweiterung der Steuerung (15) ist, die die Einstellung des Möbelstücks verwaltet.

4. Krankenhaus- oder Pflegemöbelstück nach Anspruch 1, **dadurch gekennzeichnet, dass** die Zählereinheit, die die Anzahl der Reinigungen des Möbelstücks (1) zählt, mit einer Vorrichtung zum Rücksetzen der Zählereinheit ausgestattet ist.

5. Krankenhaus- oder Pflegemöbelstück nach Anspruch 4, **dadurch gekennzeichnet, dass** die Vorrichtung zum Zurücksetzen der Zählereinheit ein Schalter auf Magnetbasis ist, der durch das Anbringen eines Magneten in seiner Gegenwart aktiviert wird.

6. Krankenhaus- oder Pflegemöbelstück nach Anspruch 5, **dadurch gekennzeichnet, dass** der Schalter auf Magnetbasis ein Hall-Sensor (19) ist.

7. Krankenhaus- oder Pflegemöbelstück nach Anspruch 4 oder 6, **dadurch gekennzeichnet, dass** die Vorrichtung zum Zurücksetzen der Zählereinheit ferner das Drücken einer Taste (17) oder das Drücken einer Tastenkombination umfasst.

8. Krankenhaus- oder Pflegemöbelstück nach Anspruch 4, **dadurch gekennzeichnet, dass** die Vorrichtung zum Zurücksetzen der Zählereinheit durch Kommunikation mit dem Mikroprozessor aktiviert wird.

9. Krankenhaus- oder Pflegemöbelstück nach Anspruch 1, **dadurch gekennzeichnet, dass** die Anzeige, dass das Möbelstück (1) gewartet werden muss, mittels Indikatoren (24) visualisiert worden ist, die als LED-Tafel konstruiert sind, die sich beispielsweise in einem Gehäuse (13) befindet, das auf dem Möbelstück (1) montiert ist.

10. Krankenhaus- oder Pflegemöbelstück nach Anspruch 1, **dadurch gekennzeichnet, dass** die Ausrüstung (13) mit einer Messvorrichtung (20) zum Messen des pH-Werts des Reinigungsmittels während des Waschens ausgestattet ist.

11. Krankenhaus- oder Pflegemöbelstück nach Anspruch 10, **dadurch gekennzeichnet, dass** die Ausrüstung (13) auf der Grundlage des pH-Wertes des Reinigungsmittels beim Waschen einen Faktor berechnet, der zusammen mit der Anzahl der Reinigungen des Möbelstücks seit der letzten Wartung des Möbelstücks (1) Teil der Bestimmung ist, wann ein Hinweis auf eine erforderliche Wartung des Möbelstücks (1) erscheint.

12. Krankenhaus- oder Pflegemöbelstück nach Anspruch 1, **dadurch gekennzeichnet, dass** die Ausrüstung (13) mit einer Zeitgebereinheit ausgestattet ist, die anzeigt, wann das Möbelstück wieder gewartet werden muss.

13. Krankenhaus- oder Pflegemöbelstück nach einem der vorhergehenden Ansprüche, **dadurch gekennzeichnet, dass** die Ausrüstung (13) Mittel zum Austausch von Daten mit anderen Einheiten in dem Möbelstück (1) aufweist, wobei andere Einheiten eine der folgenden sein können:
a. eine Steuerung (15),
b. ein Gateway, oder
c. eine Handbedienung (12)
und wo die zu übermittelnden Daten sein könnten,
d. die Anzahl der Aktivierung von Steuereinheiten,
e. Status für Zeitgeber oder Zählschaltung,
f. Status für Thermosensor,
g. Rücksetzen oder Auflösen von Zeitgebern oder Zählern,
h. Einrichten von Zeitgeber- oder Zählerschwellenwerten,
i. die Anzahl von Reinigungen seit der letzten Wartung,
j. Konfiguration der Anzeige auf der Anzeigetafel,
k. Konfiguration von Funktionen auf Tasten,
l. Konfiguration der Routine der Berechnung der pH-Messung,
m. externe Steuerung von Zeitgebern, Zählern oder Indikatoren.

14. Verfahren zum Warten von Krankenhaus- und Pflegemöbeln, ausgewählt aus der Gruppe von: einem Krankenhaus- oder Pflegebett, einem Patientenlifter, einem Behandlungstisch, einem Stuhl, basierend auf der Reinigung dieser, einschließlich eines Einleitungs- oder Rücksetzzustandes, eines Betriebszustandes mit der Anzeige des Status und eines Alarmzustandes, wobei der Einleitungszustand manuell durch Aktivierung eines Sensors, vorzugsweise eines Hall-Sensors mit einem Magneten, ausgeführt wird und einen Zähler zurücksetzt, der die Anzahl der Reinigungen zählt und den Status auf einer Anzeigetafel weiter aktualisiert, und
wo der Betriebszustand den Zähler aufrechterhält, der die Anzahl der Reinigungen des Möbelstücks zählt und
wo der Alarmzustand auftritt, wenn der Zähler, der die Anzahl der Reinigungen des Möbelstücks zählt, eine bestimmte Anzahl überschreitet, wobei der Alarmzustand auf einer Anzeigetafel angezeigt wird.

15. Verfahren zur Wartung von Krankenhaus- oder Pflegemöbeln nach Anspruch 14, worin das Krankenhaus- oder Pflegemöbel als Eingang einen pH-Sensor hat, der die Aggressivität des Reinigungsmittels misst und den Zähler mit einem Faktor manipuliert, der dem zusätzlichen Wartungsbedarf entspricht.

## Revendications

1. Élément mobilier d'hôpital ou de soin sélectionné dans le groupe constitué de:
un lit d'hôpital ou de soin, un appareil de levage de patient, une table de traitement, un fauteuil, où l'élément mobilier d'hôpital ou de soin comprend:
- au moins un dispositif d'actionnement linéaire électrique (6, 7, 8, 9) pour régler l'élément mobilier (1),
- au moins une unité de commande manuelle (12),
- au moins une commande (15) comprenant un microprocesseur, **caractérisé en ce que** l'élément mobilier d'hôpital ou de soin comprend un équipement comprenant:
- une unité formant compteur, et
- un dispositif de signalisation,
où le dispositif de signalisation étant configuré pour fournir un signal indiquant que l'élément mobilier (1) a été nettoyé est un thermo-capteur (18) qui donne un signal quand la température de l'équipement dépasse une valeur de seuil,
signal par lequel le compteur est automatiquement incrémenté et quand un nombre prédéterminé de comptes est atteint, l'unité formant compteur va indiquer un besoin d'entretien.

2. Élément mobilier d'hôpital ou de soin selon la revendication 1, **caractérisé en ce que** le signal indiquant que l'élément mobilier a été nettoyé est construit avec une hystérésis de sorte que le signal ne disparaît pas avant que la température n'ait baissé considérablement combiné avec un temps qui doit expirer.

3. Élément mobilier d'hôpital ou de soin selon la revendication 1, **caractérisé en ce que** l'équipement est une unité distincte ou une extension à la commande (15) qui gère le réglage de l'élément mobilier.

4. Élément mobilier d'hôpital ou de soin selon la revendication 1, **caractérisé en ce que** l'unité formant compteur comptant le nombre de nettoyages de l'élément mobilier (1) est équipée d'un dispositif pour remettre l'unité formant compteur à l'état initial.

5. Élément mobilier d'hôpital ou de soin selon la revendication 4, **caractérisé en ce que** le dispositif pour remettre l'unité formant compteur à l'état initial est un commutateur à base d'aimant, qui est activé en plaçant un aimant en sa présence.

6. Élément mobilier d'hôpital ou de soin selon la revendication 5, **caractérisé en ce que** le commutateur à base d'aimant est un capteur à effet Hall (19).

7. Élément mobilier d'hôpital ou de soin selon la revendication 4 ou 6, **caractérisé en ce que** le dispositif pour remettre l'unité formant compteur à l'état initial implique en outre une pression d'une touche (17) ou une pression d'une combinaison de touches.

8. Élément mobilier d'hôpital ou de soin selon la revendication 4, **caractérisé en ce que** le dispositif pour remettre l'unité formant compteur à l'état initial est activé par communication avec le microprocesseur.

9. Élément mobilier d'hôpital ou de soin selon la revendication 1, **caractérisé en ce que** l'indication que l'élément mobilier (1) a besoin d'entretien a été visualisée au moyen d'indicateurs (24) construits comme un panneau de LED situé par exemple dans un logement (13) monté sur l'élément mobilier (1).

10. Élément mobilier d'hôpital ou de soin selon la revendication 1, **caractérisé en ce que** l'équipement (13) est muni d'un appareil de mesure (20) pour mesurer la valeur de pH de l'agent de nettoyage pendant le lavage.

11. Élément mobilier d'hôpital ou de soin selon la revendication 10, **caractérisé en ce que** l'équipement (13) sur la base de la valeur de pH de l'agent de nettoyage pendant le lavage calcule un facteur qui, en même temps que le nombre de nettoyages de l'élément mobilier depuis le dernier entretien de l'élément mobilier (1), forme une partie de la détermination du moment où une indication d'entretien exigé de l'élément mobilier (1) apparaît.

12. Élément mobilier d'hôpital ou de soin selon la revendication 1, **caractérisé en ce que** l'équipement (13) est muni d'une unité formant minuterie qui indique quand l'élément mobilier a de nouveau besoin d'entretien.

13. Élément mobilier d'hôpital ou de soin selon l'une quelconque des revendications précédentes, **caractérisé en ce que** l'équipement (13) présente un moyen pour échanger des données avec d'autres unités dans l'élément mobilier (1), où d'autres unités pourraient être l'une de ce qui suit :
a. une commande (15),
b. une passerelle, ou
c. une commande manuelle (12)
et où les données à communiquer pourraient être :
d. le nombre d'activations d'unités de commande,
e. l'état pour la minuterie ou le circuit de comptage,
f. l'état pour le thermo-capteur,
g. la remise à zéro ou la réinitialisation de minuteries ou de compteurs,
h. le paramétrage de seuils de minuterie ou de compteur,
i. le nombre de nettoyages depuis le dernier entretien,
j. la configuration d'affichage sur le panneau d'indication,
k. la configuration de fonctions sur des touches,
l. la configuration de sous-programme de calcul de mesure de pH,
m. la commande externe de minuteries, de compteurs ou d'indicateurs.

14. Procédé pour l'entretien de meubles d'hôpital et de soin sélectionné dans le groupe constitué de : un lit d'hôpital ou de soin, un appareil de levage de patient, une table de traitement, un fauteuil, sur la base du nettoyage de ceux-ci, incluant un état d'initialisation ou de remise à l'état initial, une condition de fonctionnement avec indication de l'état et une condition d'alarme,
où l'état d'initialisation est effectué manuellement en activant un capteur, de préférence un capteur à effet Hall avec un aimant, et remet un compteur à l'état initial, qui compte le nombre de nettoyages et met en outre à jour l'état sur un panneau d'indication, et
où la condition de fonctionnement maintient le compteur, qui compte le nombre de nettoyages de l'élément mobilier, et
où la condition d'alarme se produit quand le compteur, qui compte le nombre de nettoyages de l'élément mobilier, dépasse un nombre déterminé, où la condition d'alarme est indiquée sur un panneau d'indication.

15. Procédé pour l'entretien de meubles d'hôpital et de soin selon la revendication 14 où les meubles d'hôpital et de soin en tant qu'entrée ont un capteur de pH, qui mesure l'agressivité de l'agent de nettoyage et manipule le compteur avec un facteur, correspondant au besoin supplémentaire d'entretien.
